# EUROPEAN PATENT SPECIFICATION

(11) **EP 1 596 726 B1**
(45) Date of publication and mention of the grant of the patent: **03.12.2008**
(21) Application number: 04705079.4
(22) Date of filing: 26.01.2004
(51) Int. Cl.: A61B 17/06, A61L 17/06, D02G 3/32

(54) **SHEATHED ELASTIC SURGIGAL THREAD**
ELASTISCHER OPERATIONSFADEN MIT HÜLLE
FILETAGE CHIRURGICAL ELASTIQUE RECOUVERT

(30) Priority: 27.01.2003 IT GE20030007
(43) Date of publication of application: 23.11.2005
(73) Proprietor: Capurro, Sergio, 16147 Genova (IT)
(72) Inventor: Capurro, Sergio, 16147 Genova (IT)
(74) Representative: Forattini, Amelia
(86) International application number: PCT/EP2004/000623
(87) International publication number: WO 2004/066847

(56) References cited:
- EP-A- 0 397 500
- EP-A- 0 485 986
- US-A- 3 890 975
- US-A- 3 926 194
- US-A- 5 080 667
- US-B1- 6 240 716

## Description

The present invention relates to a sheathed elastic thread for use in surgery, in accordance with the usual techniques, non-stretch threads are normally used in surgery, and threads that stretch by only a few millimetres when subjected to traction are considered to be elastic. Such threads are supplied in bobbins, and are subsequently cut, sterilised and mounted manually on surgical needles equipped, at the non-pointed end, with an open eye, to which the thread is secured, or are utilised in various ways for different surgical purposes.

In the most commonly used technique, the thread is fixed industrially to an atraumatic surgical needle. The atraumatic surgical needle is a needle without an eye, which, when fixed to a thread, forms a single unit with no appreciable variation in diameter. The fact that the needle has no eye means that the needle and thread cause only minimal damage on passing through the tissues. In traditional atraumatic surgical needles, the thread is fixed into a hole or groove situated at the non-pointed end of the needle. In the two-tipped atraumatic needle described in patent application No. GE 2002 A 00056 in the name of this same inventor, the thread is inserted into the needle and fixed inside it.

There exists a type of elastic thread for surgical suturing that is able to stretch by 50% to 100% or more of its original length and then to return immediately to its original length once traction ceases.

Described in patent No. EP0792622, this suturing thread is made of silicone elastomer. It is used for cutaneous expansion, before or after large excisions, and for suturing wounds prone to diastasis. The silicone elastomer thread described in patent No. EP0792622 has the following disadvantages.

A disadvantage of the silicone elastomer thread is that sometimes a fine elastic thread of 0.01- 1 mm in diameter is required, for instance to lift facial tissues or to suture the skin. However, such fine threads can easily be cut during handling with surgical instruments and may snap when pulled through the tissues by the surgeon. Indeed, if a fine silicone elastomer thread is attached by the usual technique to a surgical needle with an open eye, it is very likely to be severed.

Another disadvantage of the silicone elastomer thread, even if its diameter is greater than 1 mm, is that the silicone surface is very slippery, which makes it difficult to exert proper traction during handling with surgical gloves.

The slippery surface of the silicone elastomer thread gives rise to a further disadvantage. If used as a permanent implant for tissue lifting, according to the usual technique, when placed under elastic tension it exerts pressure exclusively on the loops at the two extremities, as the rest of the thread offers no resistance to traction. This feature of the silicone elastomer thread leads to an uneven distribution of the thread's resistance in the tissues and consequently to the risk of cutting through the tissues (cheese-wire effect). If the silicone elastomer thread were to be woven, for instance in order to repair a breach in the abdominal wall, the above disadvantage would remain. Moreover, since silicone rubber cannot be colonised by the tissues, but only encapsulated, the thread would not provide the adhesion necessary for a secure plastic repair.

In accordance with the most commonly adopted technique, normal surgical threads inserted into a hole or groove in the rear end of the traditional single-tipped atraumatic needle are held in place by pinching. However, an elastomer thread, on account of its intrinsic elasticity and its poor resistance to compression, cannot guarantee sufficient resistance to traction when secured by pinching. Moreover, even when glued to the needle, the elastomer thread cannot guarantee resistance to traction on account of the characteristics of this material.

Patent EP0960600 describes a particular patented method of fixing a silicone elastomer thread to a surgical needle. This method, however, presents the following disadvantages.

The method exploits the capacity of the extremity of the silicone elastomer thread, following polymerisation, to adhere around a length of traditional non-stretch suturing thread. In the said patent, the traditional suturing thread is fixed to the atraumatic surgical needle according to the usual techniques.

In order to form a secure bond between the two merged threads, the patented method involves marked polymerisation of the silicone rubber. This has the disadvantage of stiffening the initial length of thread, precisely in its most critical zone, where the thread needs to be flexible when drawn through the tissues. Furthermore, this joint is not as secure as a normal industrial attachment of a thread to an atraumatic surgical needle.

Another disadvantage of this method of attachment is that the silicone elastomer thread, at the point where it is fixed to the non-stretch thread connected to the needle, has a larger diameter than the needle itself. This makes it difficult for the needle/thread junction to pass through the tissues and may cause the thread to detach from the needle; alternatively, the two-tipped atraumatic needle may jerk completely out through the skin, owing to the force that the surgeon has to exert in order to overcome the resistance of the needle/thread junction.

Similar problems of inconsistency in size and poor flexibility are encountered in the other solutions proposed in the same patent EP0960600.

EP0397500 discloses a thread made of a nonabsorbable elastic core yarn (12) and of at least one absorbable relatively inelastic sheath yarn (14).

US-6240716 discloses a thread which consists of an elastic core and an external inelastic sheath, both of them nonabsorbable.

The aim of the present invention is to create an elastic thread that is flexible, resistant to traction and non-slippery. This thread must display good adhesion to the tissues when used as an implant and be able to be attached to open-eye surgical needles without the risk of snapping. In addition, it must be directly attachable to traditional atraumatic surgical needles, without requiring further polymerisation or causing noticeable variations in diameter. The said thread must be able to be woven easily in order to create the implants necessary in various surgical applications. To be used in surgery, the thread described in the present patent must be bio-compatible and cause as little inflammatory reaction as possible if left permanently in the tissues. To this end, a thread according to claim 1 has been created that consists of an elastic core and a non-elastic sheath. The core consists of one or more bio-compatible elastic threads, and the sheath of one or more bio-compatible non-stretch threads.

The non-stretch sheathing threads is> not absorbable by the organism.

The non-stretch sheathing threads are wound in a spiral fashion, with the windings set in such a way as to limit the maximum extension of the elastic thread to a precise value suited to the specific application.

The sheathed elastic thread is capable of stretching by up to 100% of its length or more and returning immediately to its original length; once the maximum extension of the sheath has been reached, the thread can be stretched no further. This thread withstands far greater traction than the same elastic thread without a sheath.

Several advantages are gained by sheathing a bio-compatible elastic thread with one or more non-stretch threads.

Firstly, the elastic thread cannot be stretched beyond its breaking point.

Secondly, the elastic thread is protected against cutting by the sharp edges of the surgical instruments with which it is handled.

Thirdly, the outer sheathing enables the thread to be gripped more easily in the hand.

Fourthly, the sheathing allows the thread to be knotted more securely.

A further advantage of the sheathed elastic thread is that it can be woven more easily than a silicone elastomer thread with a slippery surface.

Yet another advantage of the present invention is that, when a permanent implant is inserted into the tissues, the outer sheathing of the elastic thread, as created in the present invention, is colonised by the histiocytic-fibroblastic cells, which bind the thread to the tissues through which it passes; this enables the tension of the sheathed elastic thread to be distributed along its entire surface. If colonisation of the sheathing is not required, such as, for example, in the case of skin sutures that are to be removed within a few days, the surface of the sheathed elastic thread will have to be treated with special non-stick substances in accordance with standard techniques.

A further advantage of the present invention is that the sheathing of the elastic thread enables the thread to be mounted on an open-eye surgical needle without the risk of cutting the elastic core.

Moreover, the thread described in the present invention can be attached directly to a single-tipped atraumatic surgical needle. Indeed, the sheathing thread or threads can be pinched in the hole or groove at the rear end of the needle, thereby providing an adequate bond that will withstand traction. Even if the end of the thread is glued to the needle, the sheathing provides secure adhesion. When the sheathed thread is inserted into a two-tipped atraumatic needle, the risk of the thread being cut by the edges of the hole during surgical manoeuvres is considerably reduced.

The elastic core and the sheathing thread or threads can be made of natural and/or synthetic fibres. For example, the said elastic thread may be made of methylvinyl-polysiloxane silicone rubber or other bio-compatible elastic materials, such as purified natural rubber, 1,4 cis-polyisoprene, acrylonitrile butadiene copolymer, isoprene cisobutylene, conjugated ethylenepropylene/diene terpolymer or derivatives.

Among the materials used for the non-stretch non-absorbable sheathing threads, we may quote, by way of example, PTFE, polypropylene, polyurethane, polyurethane-polyether, PVDF (polyvinylidine fluoride), polyamide and derivatives, or silk and other natural fibres.

The type and windings of the non-stretch sheathing threads vary according to whether the thread is destined for use as an implant or as an elastic suture. Chemical substances and/or medications can also be incorporated into the sheathing.

The sheathing of the thread described in the present invention can be produced according to the techniques used in the textile industry in accordance with specifications and utilising materials suited to surgical applications.

The applications of the sheathed elastic thread, as described in the present invention, can be extended to permanent implants to lift the tissues and to the permanent suturing of cutaneous and subcutaneous tissues. If the sheathed elastic thread is woven, it can be used in the plastic repair of hernias, breaches in the abdominal wall, etc.

The sheathed elastic thread, as described in the present invention, can usefully substitute traditional non-stretch stitching materials in surgical operations. The advantage that the sheathed elastic thread described in the present invention has over traditional non-stretch threads, when used for suturing, is that it adapts to the swelling of the tissues, thereby maintaining firm adhesion of the tissues, and is easier to remove. When used as a permanent implant in the tissues, the sheathed elastic thread proves useful for suturing the derma or the subcutaneous or vascular tissue and for lifting facial tissues, as it counteracts drooping. In this application, it is superior to non-stretch threads in that it does not hinder the movements of the facial muscles; it neither cuts into the tissues nor forces the features to remain unnaturally static. When woven, for example into a web, the sheathed elastic thread has the advantage of creating a bio-compatible elastic surface that can be colonised by the tissues; it is therefore stable, well integrated and spreads tension over its entire surface. A web of sheathed elastic thread offers as more physiological plastic repair of hernias, for instance. Indeed, owing to its elastic and adhesive properties, it is better than webs woven from non-stretch threads at adapting to sudden increases in endoabdominal pressure, which may cause a recurrence of the hernia.

All of the above-mentioned advantages, and many others, will be seen from the description of the following figures, which are attached by way of illustration and are not regarded as limiting.

Fig. 1 depicts a sheathed elastic thread as described in the present invention;
Fig. 2 depicts a sheathed elastic thread with a reduced diameter at one end;
Fig. 3 shows a sheathed elastic thread inserted into the hole or groove of a single-tipped atraumatic needle;
Fig. 4 shows a sheathed elastic thread inserted into a two-tipped atraumatic needle;
Fig. 5 shows a sheathed elastic thread woven into a web;

In the various figures, the same elements are indicated by the same numbers.

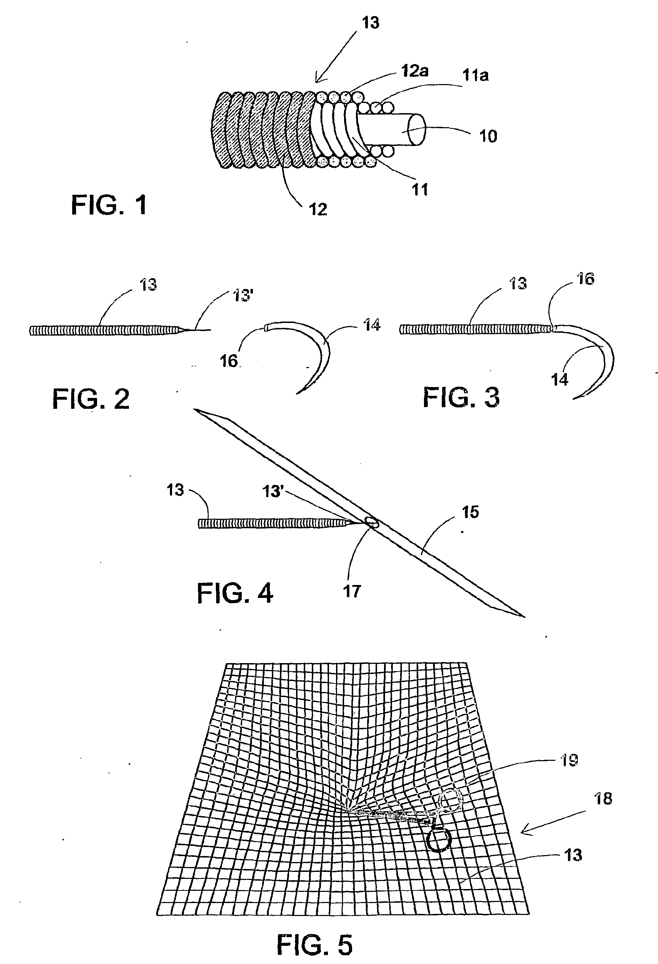

In Figure 1, a sheathed elastic thread (13) as described in the present invention is illustrated by way of example.

The core (10) of the thread is made of bio-compatible elastic material capable of stretching by up to 100% of its length or more and of returning immediately to its original length.

The elastic core (10) is sheathed by a non-stretch thread (11) wound in a spiral fashion, for example anti-clockwise, the windings of which, seen in section, are indicated (11a).

The spiral of the non-stretch thread (11) is covered by a second outer thread (12) wound in a spiral in the opposite direction, for example clockwise, the windings of which, seen in section, are indicated (12a).

In order to facilitate insertion of the thread (13) into a needle (14), the diameter of one end of the thread (13) is reduced by stretching this portion of the thread to its maximum. The thinned end (13') is then fixed by means of a bio-compatible glue, as illustrated in Figure 2. Alternatively, a low-temperature treatment can be used to maintain the thinning of the end (13') of the thread.

In Figure 2, a normal atraumatic suturing needle (14) with an axial hole (16) at the non-pointed end is seen in front of the thread (13).

In Figure 3, the sheathed elastic thread (13) is shown with the thinned end (13') inserted into the hole (16) of the needle (14). The thread (13) can be attached to the needle (14) in the usual ways, by compressing the non-stretch threads (11 and 12) of the sheathing, comprising or not comprising the elastic core (10).

Another recommended method of attaching the thread (13) to the needle (14) is to use bio-compatible glue.

The end of the thread (13) that is not attached to the needle (14) can also be treated with a glue that has suitable characteristics of bio-compatibility and resistance to sterilisation, in order to prevent the sheathing from fraying.

Obviously, production of the materials used in the sheathed elastic thread (13) and attachment of the thread to the needle (14) must take place in a controlled environment and in conformity with current regulations.

Figure 4 shows the sheathed elastic thread (13) with its thinned end (13') inserted into the hole (17) of a two-tipped atraumatic needle (15) by means of the modalities described in the above-mentioned patent application N. GE 2002 A 00056.

Figure 5 depicts a sheathed elastic thread woven into a web.

Traction with forceps (19) illustrates the elasticity and pliability of the web (18), owing to the elastic features of the sheathed thread as described in the present invention.

The present invention comprises all those variations in detail and all modifications that may prove obvious to a technician in this field, and which do not lie outside the ambit of the present invention, but which are understood to be included within the area of the following claims.

## Claims

1. Surgical thread (13), comprising an elastic core (10) and a non-elastic sheathing, wherein the elastic core (10) consists of one or more bio-compatible elastic threads, and the sheathing consists of one or more non-stretch threads (11, 12) made of bio-compatible material, said non-stretch sheathing thread or threads (11, 12) and said elastic core (10) being made of non-absorbable material, said non-stretch sheathing thread (11) being wound in a spiral fashion, forming overlying spirals wound in opposite directions, with the windings to limit the maximum extension of the elastic thread.

2. Surgical thread (13), according to claim 1, **characterised in that** it comprises an elastic core (10) made of methylvinyl-polysiloxane silicone rubber or other bio-compatible elastic materials, such as purified natural rubber, 1,4 cis-polyisoprene, acrylonitrile butadiene copolymer, isoprene cisobutylene, conjugated ethylene-propylene/diene terpolymer.

3. Surgical thread (13), according to claim 1, **characterised in that** said non-absorbable material used in the non-stretch sheathing thread or threads (11, 12) is constituted by PTFE or polypropylene, polyurethane, polyurethane-polyether, PVDF (polyvinylidine fluoride), polyamide or derivatives of these, or by silk or other natural fibres.

4. Surgical thread (13), according to claim 1, which is woven in order to create an elastic surface for implantation.

5. Process to attach the surgical thread (13), according to claim 1, to a needle (14, 15), **characterised in that** the end portion of the thread (13) is thinned by applying maximum tension, and that such thinning of the end (13') is maintained by means of low-temperature treatment.

## Patentansprüche

1. Chirurgischer Faden (13), der einen elastischen Kern (10) und eine nicht elastische Ummantelung umfasst, wobei der elastische Kern (10) aus einem oder mehreren biologisch verträglichen elastischen Fäden besteht und die Ummantelung aus einem oder mehreren dehnungsfreien Fäden (11, 12) besteht, die aus einem biologisch verträglichen Material hergestellt sind, wobei der dehnungsfreie Ummantelungsfaden oder die Fäden (11, 12) und der elastische Kern (10) aus einem nicht absorbierbaren Material hergestellt sind, wobei der dehnungsfreie Ummantelungsfaden (11) auf eine spiralige Weise gewickelt ist, wobei er übereinanderliegende Spiralen bildet, die in entgegengesetzten Richtungen gewickelt sind, wobei die Windungen dazu dienen, die maximale Ausdehnung des elastischen Fadens zu begrenzen.

2. Chirurgischer Faden (13) nach Anspruch 1, **dadurch gekennzeichnet, dass** er einen elastischen Kern (10) umfasst, der aus Methylvinyl-Polysilexan-Silikonkautschuk oder anderen biologisch verträglichen Materialien, wie beispielsweise gereinigtem Naturkautschuk, 1,4-Cis-Polyisopren, Acrylnitril-Butadien-Copolymer, Isopren-Cisobutylen, konjugiertem Ethylen-Propylen-/Dien-Terpolymer, hergestellt ist.

3. Chirurgischer Faden (13) nach Anspruch 1, **dadurch gekennzeichnet, dass** das nicht absorbierbare Material, das bei dem dehnungsfreien Ummantelungsfaden oder den Fäden (11, 12) verwendet wird, durch PTFE oder Polypropylen, Polyurethan, Polyurethan-Polyether, PVDF (Polyvinylidinfluorid), Polyamid oder Derivate derselben oder durch Seide oder andere Naturfasern dargestellt wird.

4. Chirurgischer Faden (13) nach Anspruch 1, der gewebt ist, um eine elastische Oberfläche zum Implantieren zu erzeugen.

5. Verfahren zum Befestigen des chirurgischen Fadens (13) nach Anspruch 1 an einer Nadel (14, 15), **dadurch gekennzeichnet, dass** der Endabschnitt des Fadens (13) durch Anwenden einer maximalen Spannung dünner gemacht wird und dass ein solches Dünnermachen des Endes (13') mit Hilfe einer Tieftemperaturbehandlung aufrechterhalten wird.

## Revendications

1. Fil chirurgical (13), comprenant un centre élastique (10) et un gainage non élastique, dans lequel le centre élastique (10) consiste en un ou plusieurs fils élastiques biocompatibles, et le gainage consiste en un ou plusieurs fils non extensibles (11, 12) constitués d'un matériau biocompatible, ledit ou lesdits fil(s) de gainage non extensible(s) (11, 12) et ledit centre élastique (10) étant constitués d'un matériau non absorbable, ledit fil de gainage non extensible (11) étant enroulé en spirale, formant des spirales de recouvrement s'enroulant dans des directions opposées, les enroulements limitant l'extension maximale du fil élastique.

2. Fil chirurgical (13), selon la revendication 1, **caractérisé en ce qu'**il comprend un centre élastique (10) constitué de caoutchouc méthylvinyl-polysiloxane silicone ou d'autres matériaux élastiques biocompatibles, tels que le caoutchouc naturel purifié, le 1,4 cis-polyisoprène, un copolymère acrylonitrile butadiène, l'isoprène cisobutylène, un terpolymère conjugué éthylène-propylène/diène.

3. Fil chirurgical (13), selon la revendication 1, **caractérisé en ce** ledit matériau non absorbable utilisé dans le ou les fil(s) de gainage non extensible(s) (11, 12) est constitué de PTFE ou de polypropylène, polyuréthane, polyuréthane-polyéther, PVDF (polyvinylidine fluorure), polyamide ou de dérivés de ceux-ci, ou de soie ou d'autres fibres naturelles.

4. Fil chirurgical (13), selon la revendication 1, qui est tressé de manière à créer une surface élastique pour implantation.

5. Procédé pour fixer le fil chirurgical (13), selon la revendication 1, à une aiguille (14, 15), **caractérisé en ce que** l'extrémité du fil (13) est amincie par application d'une tension maximale, et qu'un tel amincissement de l'extrémité (13') est conservé au moyen d'un traitement à basse température.
